# EUROPÄISCHE PATENTSCHRIFT

(11) **EP 1 339 705 B1**
(45) Veröffentlichungstag und Bekanntmachung des Hinweises auf die Patenterteilung: **07.06.2006**
(21) Anmeldenummer: 01990455.6
(22) Anmeldetag: 29.11.2001
(51) Int. Cl.: C07D 339/04

(54) **VERFAHREN ZUR HERSTELLUNG VON LIPONSÄURE UND DIHYDROLIPONSAÜRE**
METHOD FOR PRODUCING LIPOIC ACID AND DIHYDROLIPOIC ACID
PROCEDE DE FABRICATION D'ACIDE LIPONIQUE ET D'ACIDE DIHYDROLIPONIQUE

(30) Priorität: 30.11.2000 DE 10059718
(43) Veröffentlichungstag der Anmeldung: 03.09.2003
(73) Patentinhaber: BASF Aktiengesellschaft, 67056 Ludwigshafen (DE)
(72) Erfinder: KLATT, Martin, Jochen, 67098 Bad Dürkheim (DE); NIEBEL, Markus, 68163 Mannheim (DE); PAUST, Joachim, 67141 Neuhofen (DE)
(86) Internationale Anmeldenummer: PCT/EP2001/013955
(87) Internationale Veröffentlichungsnummer: WO 2002/044163

(56) Entgegenhaltungen:
- EP-A- 0 487 986
- BRINGMANN G ET AL: "A SHORT AND PRODUCTIVE SYNTHESIS OF (R)-ALPHA-LIPOIC ACID" ZEITSCHRIFT FUR NATURFORSCHUNG, TEIL B: ANORGANISCHE CHEMIE, ORGANISCHE CHEMIE, VERLAG DER ZEITSCHRIFT FUR NATURFORSCHUNG. TUBINGEN, DE, Nr. 54B, 1999, Seiten 655-661, XP001063331 ISSN: 0932-0776 in der Anmeldung erwähnt

## Beschreibung

Dihydroliponsäure und Liponsäure sind natürlich vorkommende Substanzen, denen eine besondere Bedeutung im Zellstoffwechsel zukommt. R-Liponsäure spielt als Coenzym, z.B. der Pyruvatdehydrogenase, eine zentrale Rolle bei der Energiegewinnung. R-Liponsäure wird zur vollen Entfaltung ihrer sehr guten antioxidativen Eigenschaften zu Dihydroliponsäure im Stoffwechsel aktiviert. R-Liponsäure beeinflußt positiv altersbedingte Veränderungen im Stoffwechsel und ist daher auch im kosmetischen Bereich von Interesse.

Aus der Literatur
Bringmann, Z. Naturforschung 1999, 54b, 665-661;
Adger, Bioorg. Med. Chem. 1997, 5, 253-61;
Yadav, J. Scientific & Industrial Res. 1990, 49, 400-409; Gopalan, Tetrahedron Lett 1989, 42, 5705;
Rao, Synth. Commun. 17, 1987a, 11, 1339-1347
Rao, Tetahedron Lett. 28, 1987b, 19, 2183-2186
Brookes, Perkin Transaction I, 1988, 9-12;
Brookes, Chemical Communication 1983, 1051-53; und
JP 1960-35704; EP 543088; EP 487 986;
sind verschiedene Methoden der Herstellung von optisch reiner R- und S-Liponsäure bzw. Dihydroliponsäure bekannt.

So werden die enantiomerenreine Liponsäure und Dihydroliponsäure auf verschiedenen Wegen wie chemische oder enzymatische Spaltung des Razemats, mit der Hilfe von chiralen Templaten, durch enantioselektive Synthese oder mikrobiologische Transformation hergestellt.

Im folgenden werden die Synthesen von R-Liponsäure und R-Dihydroliponsäure beispielhaft beschrieben. Analog können auch jeweils die S-Enantiomeren hergestellt werden.

Bei Bringmann et al. werden zwei Synthesewege für R-Liponsäure vorgestellt, die von chiralen 6,8-Dihydroxyoctansäureestern (1) 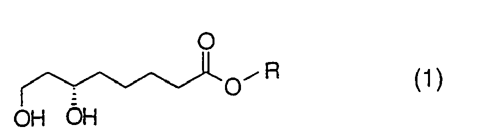 ausgehen.

Die Ausbeuten von Liponsäure bezüglich (1) liegen bei 65 %; das erhaltene Material besitzt bei der Schwefel-Einführung mit KSAc aber nur eine Reinheit im GC von 98 %, die für humane Anwendungen problematisch sein könnte.

Alternativ kann laut Bringmann et al. die Schwefeleinführung in DMF mit NaS+S geschehen, wobei die anschließende Verseifung mit Lipase oder Kaliumcarbonat geschehen kann. Der intermediär anfallende Liponsäuremethylester ist sehr polymerisationsempfindlich.

Rao, 1987a, und 1987b beschreiben die Schwefeleinführung in das Mesylat des 6,8-Dihydroxyoctansäureester (1) mit NaS+S in DMF mit einer Ausbeute von 70 %.

Die publizierten Synthesen gehen entweder über viele Schritte und/oder verwenden teure Ausgangsprodukte oder Reaktionsbedingungen. Unter Ausbeute-, Umwelt- und/oder Kostenüberlegungen sind die bekannten Verfahren verbesserungswürdig. Da Liponsäure und Dihydroliponsäure auch am Menschen eingesetzt werden sollen, sind möglichst reine Produkte erwünscht, die in hohen Ausbeuten einfach hergestellt werden können.

Das technische Problem, das der vorliegenden Erfindung somit zugrunde liegt, ist daher ein Verfahren bereitzustellen, durch das Liponsäure und Dihydroliponsäure in möglichst hohen Ausbeuten und in hoher Reinheit ökonomisch und ökologisch vorteilhaft erhalten werden kann.

Die Lösung des technischen Problems wird durch die in den Ansprüchen beschriebenen Anwendungsformen bereitgestellt.

Folglich betrifft die vorliegende Erfindung ein Verfahren zur Herstellung von Dihydroliponsäure, umfassend
(a) Umsetzung von 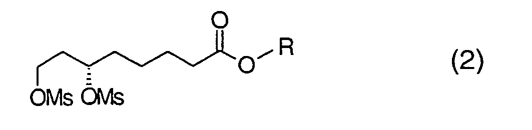 wobei
MS für SO₂-R' steht und R und R' unabhängig voneinander C₁-C₆-Alkyl, C₃-C₈-Cycloalkyl, C₃-C₈-Cycloalkylalkyl, Aryl oder Aralkyl bedeutet,
mit Natriumsulfid und Schwefel in Methanol.

Die Verbindung (2) wird z.B. durch Umsetzung des entsprechenden 6,8-Dihydroxyoctansäurealkylester (1) mit Triethylamin und Mesylchlorid hergestellt. Die bevorzugten Alkylester sind C₁-C₆-Alkyl, besonders bevorzugt ist Methyl.

Aryl oder Ar in Aralkyl bedeutet bevorzugt Phenyl, Naphthyl, das jeweils mit ein, zwei oder drei C₁-C₄-Alkylresten substituiert sein kann; "alkyl" in Aralkyl oder Cycloalkyl-alkyl bedeutet bevorzugt C₁-C₄-Alkyl, besonders bevorzugt -CH₂-.
Die bevorzugte Bedeutung für Ms ist Mesylat oder Tosylat.

Überraschenderweise führt die Verwendung von Natriumsulfid und Schwefel in Methanol zur Schwefeleinführung in (2) zu einer im Vergleich zu DMF wesentlich höheren Ausbeute und Reinheit. So wird bei der im Stand der Technik beschriebenen Verwendung von Natriumsulfid in DMF zur Schwefeleinführung in (2) nur eine Ausbeute von 70 % bis 75 % (Rao, 1987a; Beispiele) erreicht. Durch das erfindungsgemäße Verfahren wird eine hohe chemische Reinheit von R- oder S-Dihydroliponsäure erreicht. Vorteilhafterweise kann jedoch nicht nur die Ausbeute und Reinheit der Produkte durch die Verwendung von Methanol als Lösungsmittel bei der Einführung von Schwefel verbessert werden, sondern zudem auch die Herstellung von Dihydroliponsäure nach dem erfindungsgemäßen Verfahren vereinfacht und eine kostensparende Produktion ermöglicht werden: Methanol ist ein preisgünstigeres Lösungsmittel als DMF.

Überraschender Weise eignet sich das erfindungsgemäße Verfahren auch für die Schwefeleinführung in andere Verbindungen.

Folglich betrifft die Erfindung auch ein Verfahren zur Herstellung von Verbindungen, enthaltend das Strukturelement (3) 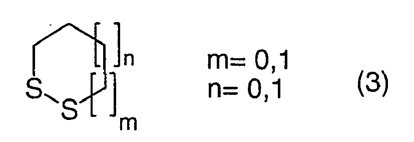 umfassend die
(a) Umsetzung von (4) 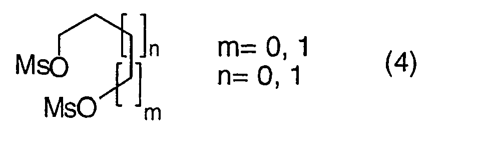
mit Natriumsulfid-Trihydrat und Schwefel in Methanol.

In einer Ausführungsform besitzt das Strukturelement (3) die Substituenten R¹, R², R³ und R⁴ (Verbindung (3a)): 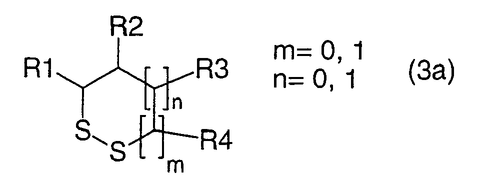 wobei,
- R¹, R², R³, und/oder R⁴,: unabhängig voneinander sein kann: H; unverzweigtes oder verzweigtes C₁-C₂₀-Alkyl, wobei 0 bis 3 Kohlenstoff-Atome ersetzt sein können durch O, S, NZ, und/oder -X¹-(C=X²)-, mit X¹ gleich Bindung, O, S oder NZ, und/oder X² gleich O, S, oder NZ; und/oder mono-, bi-, oder tricyclischer, aromatischer, gesättigter, oder teilweise ungesättigter C₀-C₆-Alkyl-Carbo- oder Heterozyklus mit 3 bis 17 Kohlenstoffatomen, wobei 0 bis 3 Heteroatome ausgewählt aus S, N, und/oder O sein können; und wobei jedes Kohlenstoffatom der Alkylketten oder des Rings bis zu drei der folgenden Substituenten OZ, SZ, (C=O)-OZ, NZZ¹, C₁ bis C₆-Alkyl tragen kann; und wobei Z und/oder Z¹ gleich H oder C₁-C₆-Alkyl sein kann.

Vorzugsweise gilt n+m gleich 1 oder 2.

Das erfindungsgemäße Verfahren eignet sich auch zur Herstellung von Derivaten der Liponsäure, Dihydroliponsäure und/oder von (3), wie sie z.B. in DE 41 37 773, DE 43 43 592, DE 43 43 593, EP 812 590, WO 00/24734, WO 00/59899 und WO 00/53601 beschrieben sind und die hier mit umfasst sind. Insbesondere sind auch die Salze, Ester oder Amide der hier genannten Verbindungen, vorzugsweise der Dihydroliponsäure, Liponsäure, oder der Verbindung (3), oder der in der genannten Literatur aufgelisteten Verbindungen umfasst. Ebenfalls umfasst sind Verfahren zur Herstellung von Metaboliten der Liponsäure oder Dihydroliponsäure, wie z.B. Bisnorliponsäure oder Tetraliponsäure.

Die erfindungsgemäße Umsetzung der Sulfonsäure-Derivate (2), z.B. des Mesylats, erfolgt bevorzugt in einer Na₂S-S-Mischung in Methanol. Unter dem Begriff "Methanol" werden erfindungsgemäß methanolische Lösungen verstanden, in denen sich Natriumsulfid, vorzugsweise das Trihydrat, und Schwefel gut lösen. Wie hoch der Anteil eines oder mehrerer anderen/r Lösungsmittel an der methanolischen Lösung ist, weiß der Fachmann und hängt von dem/den "anderen Lösungsmittel(n)" ab. Unter "anderes/n Lösungsmittel(n)" werden z.B. Wasser, DMF, oder andere Alkohole, z.B. Ethanol, Isopropanol, etc. verstanden. Der Fachmann kann durch eine dem Stand der Technik entsprechenden Testreihe leicht testen, ob eine methanolische Lösung für das erfindungsgemäße verfahren geeignet ist, insbesondere ob sich darin Natriumsulfid, vorzugsweise Natriumsulfid-Trihydrat, und Schwefel gut lösen. Folglich besteht die methanolische Lösung aus mindestens 80 % Gew.-Gehalt Methanol, mehr bevorzugt sind 90 % Gew.-Gehalt, besonders bevorzugt 95 % Gew.-Gehalt Methanol, am meisten bevorzugt ist mehr als 95 % Methanol. Bevorzugter Zusatz ist Wasser und/oder Ethanol. Die methanolische Mischung enthält bevorzugt mindestens äquimolare Mengen an Na₂S, S und Mesylat und bevorzugt einen geringeren als einen je 100 %igen molaren Überschuss an Na₂S und S bezogen auf Mesylat. Mehr bevorzugt ist ein 25- bis 35 %iger molarer Überschuß an Na₂S und ein 45- bis 55 %iger molarer Überschuß an Schwefel über dem Mesylat. Die methanolische Na₂-S-Mischung wird bevorzugt vorher aufgekocht.

In einer bevorzugten Ausführungsform wird das erfindungsgemäße Verfahren durch Umsetzung mit Natriumsulfid-Trihydrat durchgeführt. Überraschenderweise ist die Verwendung von Natriumsulfid mit einem geringen Kristallwassergehalt besonders vorteilhaft und führt in dem erfindungsgemäßen Verfahren zu einer sehr hohen Ausbeute. Besonders vorteilhaft ist der Einsatz des Trihydrats gegenüber bisher in der Literatur eingesetztem Nonahydrat bzw. auch gegenüber wasserfreiem Natriumsulfid. Es zeigte sich, dass Natriumsulfid-Trihydrat zur höchste Ausbeute an Diydroliponsäure führt.

In einer weiteren Ausführungsform liegt in dem erfindungsgemäße Verfahren Schwefel in einem molaren Überschuss über dem Natriumsulfid, insbesondere dem Natriumsulfid-Trihydrat, vor. In einer bevorzugten Ausführungsform wird ein molarer Überschuss von 5 bis 30 % an Schwefel über Natriumsulfid verwendet. Vorzugsweise liegt ein Überschuss an Natriumsulfid und Schwefel über dem Mesylat vor. So kann in einer besonders bevorzugten Ausführungsform 1,3 Äquivalente Natriumsulfid und 1,5 Äquivalente elementarer Schwefel bezüglich 1,0 Äquivalente Mesylat in Methanol verwendet werden.

In einer weiteren Ausführungsform umfasst das erfindungsgemäße Verfahren mindestens einen weiteren anschließenden Verfahrensschritt, ausgewählt aus der Gruppe bestehend aus:
(b) Umsetzung mit einem komplexen Hydrid;
(c) Extraktion einer protischen Lösung R-Dihydroliponsäure oder S-Dihydroliponsäure mit organischen Lösungsmitteln bei einem pH-Wert von 9 bis 10;
(d) Extraktion von R-Dihydroliponsäure oder S-Dihydroliponsäure mit organischen Lösungsmitteln aus einer protischen Lösung bei einem pH-Wert von 4 bis 5; und
(e) Destillation der Dihydroliponsäure.

Unter "komplexen Hydriden" werden bevorzugt Boranate verstanden, insbesondere Alkaliboranate wie NaBH₄. Die Umsetzung mit komplexen Hydriden erfolgt bevorzugt in alkalischer Lösung, besonders in konzentrierter Alkalihydroxid-Lösung. Besonders bevorzugt ist eine Borol-Lösung (z.B. 12 %ig NaBH₄ in 14M NaOH; die jeweilige Zusammensetzung der Borol-Lösung kann je nach Hersteller und Charge varieren).

Wird nach der Umsetzung der mit einem komplexen Hydrid die protische Lösung von Dihydroliponsäure bei einem pH-Wert von 9 bis 10, bevorzugt bei ungefähr 9,5, mit einem organischen Lösungsmittel extrahiert, erhält man nach Aufarbeitung zu Liponsäure mehr Ausbeute an Kristallisat.

Unter protischen Lösungen werden Lösungsmittelgemische mit mindestens 30 % Wasser, bevorzugt mehr als 50 % Wasser, besonders bevorzugt mehr als 75 % Wasser, verstanden. Die andere Komponente sind polare Lösungsmittel wie DMF oder Alkohole, insbesondere Methanol. Organische Lösungsmittel für die Extraktion sind bevorzugt apolare Lösungsmittel, z.B. halogenierte Lösungsmittel wie Methylenchlorid oder Chloroform, Glykolether, Ether wie Diethylether oder Methyl-t.-butylether, Ester wie Essigester, aliphatische und aromatische Kohlenwasserstoffe wie Cyclohexan, Hexan, Heptan, Toluol, oder deren Gemische, wobei als Lösungsmittel Hexan, Heptan, Toluol und Essigester bevorzugt sind.

Wird die protische Lösung von Dihydroliponsäure bei einem pH-Wert von 4 bis 5, bevorzugt bei ungefähr 4,5 in organisches Lösungsmittel extrahiert, erhält man nach Aufarbeitung zu Liponsäure mehr Ausbeute an Kristallisat. An diese Schritte kann eine Destillation der Dihydroliponsäure angeschlossen werden.

Überraschenderweise lässt sich Dihydroliponsäure ohne wesentliche Zersetzung in einem Temperaturbereich von 160 bis 220°C, bevorzugt sogar bei 180 bis 210°C, besonders bevorzugt bei 200°C ± 5°C, bei Drücken von 0,5 bis 5 mbar, besonders bevorzugt bei 1 bis 3 mbar, destillieren. Die Destillation wird bevorzugt kontinuierlich durchgeführt (Sambay, Fallfilm- oder Dünnschichtverdampfer). Dieser Druckbereich ist technisch ohne erheblichen Aufwand zu realisieren. Überraschenderweise erhält man nach anschließender Oxidation und Kristallisation über 10 % mehr Liponsäure aus der Dihydroliponsäure als ohne Destillation. Diese weitere Optimierung der Reinigung der Dihydroliponsäure führte überraschenderweise, obwohl mehr Schritte eingeführt werden, zu höheren Ausbeuten an reiner Liponsäure. Überraschenderweise wurde auch gefunden, dass die Umkehr der Extraktionsschritte (erst Extraktion bei pH 4 bis 5 und anschließende Reinigung bei pH 9 bis 10) auch ohne Destillation der Dihydroliponsäure hohe Ausbeuten an Liponsäurekristallisat möglich macht. Diese Verfahrensweise ist ebenfalls besonders bevorzugt.

Bevorzugt wird das erfindungsgemäße Verfahren ohne Isolierung der Zwischenprodukte durchgeführt.

In einer weiteren bevorzugten Ausführungsform wird in dem erfindungsgemäßen Verfahren Natriumsulfid-Trihydrat und Schwefel in Methanol zu (2) oder (4) zugegeben. Überraschenderweise lassen Natriumsulfid-Trihydrat und Schwefel sich bei Raumtemperatur leicht lösen in Methanol und ergeben eine klare, leicht dosierbare Flüssigkeit. Somit kann vorteilhafter Weise das erfindungsgemäße Verfahren "invers" gefahren werden. Unter dem Begriff "invers" wird hier verstanden, dass das Schwefelreagenz aus Natriumsulfid-Trihydrat und Schwefel in Methanol, vorzugsweise unter einer Schutzatmosphäre, z.B. N₂, direkt zu dem Mesylat (2) oder (4) dosiert wird. Dieses besonders bevorzugte Verfahren führt zu einer verringerten Verunreinigung der Dihydroliponsäure. Die "inverse" Fahrweise bedingt zudem eine Ausbeutesteigerungen für die Herstellung von Liponsäure auf 85 %. Verfahrenstechnisch bietet die inverse Fahrweise zusätzlich den Vorteil, dass das zuvor hergestellte Mesylat nicht mehr aus dem Kessel abgelassen werden muss. Die Schwefeleinführung kann als Eintopfreaktion durchgeführt werden. Dem Fachmann ist bei der Durchführung des erfindungsgemäßen Verfahren klar, dass die Ausbeute und das Auftreten von Nebenprodukten von der Art der Dosierung abhängt. So kann sowohl eine zu schnelle als auch eine zu langsame Dosierung zur verstärkten Bildung von Nebenprodukten führen. Der Fachmann weiß die jeweiligen Dosierungsgeschwindigkeiten und -art an die verwendeten Reaktionstemperaturen, Volumina, die bevorzuge Produktqualität, oder die Art Durchmischung anzupassen.

In einer bevorzugten Ausführungsform liegt die Reaktionstemperatur für das erfindungsgemäße Verfahren zwischen Raumtemperatur und Rückfluss. Besonders bevorzugt ist eine Reaktionstemperatur die zwischen 35 und 45°C liegt. Am meisten bevorzugt ist eine Reaktionstemperatur von 40°C. Es wurde gefunden, dass unter bestimmten Reaktionsbedingungen eine Reaktionstemperatur von 40°C zur höchsten Ausbeute führt. Bei zu geringen Temperaturen kann z.B. keine vollständige Umsetzung erreicht werden. Der Fachmann weiß die Reaktionstemperatur an die jeweiligen Verfahrensbedingungen anzupassen.

In einer besonders bevorzugten Ausführungsform betrifft das erfindungsgemäße Verfahren die Herstellung von R-Liponsäure oder S-Liponsäure wobei das Verfahren eine Oxidation der R- oder S-Dihydroliponsäure umfasst. Wird der Ansatz nach Darstellung der Dihydroliponsäure angesäuert (z.B. pH <2) und mit einem organischen Lösungsmittel (bevorzugt Essigester oder Toluol) extrahiert, erhält man eine hohe Ausbeute Dihydroliponsäure. Wird die so erhaltene Dihydroliponsäure zu Liponsäure oxidiert und kristallisiert, erhält man in hoher Ausbeute sehr reine Liponsäure (GC > 99,5 %, ee HPLC (CSP) > 99 % (Nachweisgrenze)). Die Oxidation kann z.B. mit FeCl₃/Luft erfolgen, die Kristallisation bevorzugt in Heptan/Toluol (WO 00/08012).

Die Schritte in oben angegebenen Verfahren zur Aufreinigung von Dihydroliponsäure führen einzeln und in Kombination zu höheren Ausbeuten an kristallisierter Liponsäure. Bevorzugt ist die Kombination einzelner Schritte, ganz besonders bevorzugt ist das Durchführen aller o.g. Verfahrensschritte, insbesondere in der Reihenfolge wie in den Beispielen angegeben.

Das erfindungsgemäße Verfahren umfasst auch die Herstellung von R-Dihydroliponsäure oder S-Dihydroliponsäure, wobei die R-Dihydroliponsäure oder S-Dihydroliponsäure chemisch rein ist. Vorzugsweise wird auch die Herstellung chemisch reiner Dihydroliponsäure umfasst. Unter chemisch reiner Liponsäure oder reiner Dihydroliponsäure wird chemisch und insbesondere enantiomerenreine Liponsäure bzw. Dihydroliponsäure verstanden. Unter chemisch reiner R- Dihydroliponsäure bzw. S-Dihydroliponsäure und R-Liponsäure bzw. S-Liponsäure wird Material verstanden, das bevorzugt eine Enantiomerenreinheit (ee-Wert bestimmt mit HPLC, CSP, vorzugsweise gemäß dem in EP 694 542 beschriebenen Verfahren) von 70 %, bevorzugt 80 %, besonders bevorzugt 90 %, ganz besonders bevorzugt 95 %, noch mehr bevorzugt 97 % oder 98 %, am meisten bevorzugt 99 % und größer, d.h. am Detektionslimit liegend, besitzt. Besonders gewünscht ist bezüglich der chemischen Reinheit (GC oder HPLC) von R- bzw. S-Dihydroliponsäure Material mit einer Reinheit größer gleich 80 %, besonders bevorzugt größer gleich 90 %, ganz besonders bevorzugt größer gleich 95 % bzw. 97 %. Bezüglich der chemischen Reinheit von R- oder S-Liponsäure ist Material bevorzugt mit größer 99 %, besonders bevorzugt größer 99,5 %, ganz besonders bevorzugt größer 99,9 %. Dies entspricht dem Detektionslimit der verwendeten Methoden.

Das erfindungsgemäße Verfahren umfassend auch die Weiterverarbeitung von R-Liponsäure oder S-Liponsäure in pharmakologisch verträgliche Salze, oder Derivate. Außerdem betrifft die Erfindung die Weiterverarbeitung von R-Liponsäure oder S-Liponsäure nach dem erfindungsgemäßen Verfahren in pharmakologisch verträgliche Derivate wie Ester oder Amide der Liponsäure. Des weiteren betrifft die Erfindung auch die Weiterverarbeitung der erfindungsgemäß hergestellten R- oder S-Liponsäure in pharmakologisch verträgliche Salze, wie Alkali- und Erdalkalisalze. Ebenso betrifft das erfindungsgemäße Verfahren die Herstellung von Metaboliten, wie z.B. Bosnorliponsäure oder Tetranorliponsäure sowie deren Salze, Ester oder Amide. Die Umsetzung und andere Derivate sind aus der Literatur bekannt, z.B. in DE 43 43 592, 43 43 593, EP 812 590, WO 00/24734, WO 00/59899, WO 00/53601.

Die Erfindung betrifft auch ein Verfahren zur Herstellung von Kosmetik, eines Arzneimittels oder Pharmakon, umfassend einen der Schritte des erfindungsgemäßen Verfahrens und,
(g) Formulierung der R-Dihydroliponsäure, S-Dihydroliponsäure, R-Liponsäure oder S-Liponsäure in einer pharmakologisch oder dermatologisch verträglichen Form.

Liponsäure und Dihydroliponsäure können auch als Nutraceutical im Lebensmittelbereich eingesetzt werden. Auch ein Einsatz in Kosmetik, als Arzneimittel oder Pharmakon von Dihydroliponsäure und/oder Liponsäure ist möglich. Bekannt ist, daß R-Liponsäure die Insulinsensitivität erhöht und damit als Antidiabetikum, auch für die Verhinderung und Linderung von diabetischen Spätschäden, verwendet werden kann. Weiterhin kann Liponsäure oder Dihydroliponsäure oder Derivate zur Behandlung von Glukosestoffwechselstörungen (z.B. ZNS), bei Insulinresistenz, Krebs und bei Hörstörungen eingesetzt werden.

Des weiteren betrifft die Erfindung eine Lösung enthaltend Natriumsulfid-Trihydrat und Schwefel in Methanol, wobei der Schwefel in einem molaren Überschuss vorliegt. Unter dem Begriff "Methanol" werden erfindungsgemäß methanolische Lösungen wie oben definiert verstanden. Die methanolische Mischung enthält bevorzugt mindestens äquimolare Mengen an Na₂S, und S. Die erfindungsgemäße Lösung, die durch Lösen von Na₂S • 3 H₂O und elementarem Schwefel in Methanol bei Raumtemperatur hergestellt werden kann, bietet im Vergleich zum Stand der Technik wesentliche Vorteile: Das Reagenz ist eine klare, gut dosierfähige Lösung. Es ermöglicht die "inversen Fahrweise", bei der erstmals das Schwefelreagenz zum Mesylat (2) oder (4) dosiert werden kann, was zur Reduktion der Oligomerenbildung führen kann. Durch die Verwendung der Lösung kann die Schwefeleinführung auch bei niedrigen Temperaturen, z.B. bei 40°C (früher 65°C oder höher) durchgeführt werden. Der Einsatz der erfindungsgemäßen Lösung führt zudem zu einer verfahrenstechnische Vereinfachung, da das zuvor hergestellte Mesylat im Kessel verbleiben kann. Aufgrund der erfindungsgemäßen Lösung kann die Schwefeleinführung kann als "Eintopfreaktion" durchgeführt werden.

In einer besonders bevorzugten Ausführungsform liegt der molare Schwefelüberschuss der erfindungsgemäßen Lösung zwischen 5 und 30 % gegenüber dem Natriumsulfid-Trihydrat. In einer bevorzugten Ausführungsform wird die erfindungsgemäße Lösung so eingesetzt, dass das Verhältnis von Mesylat, Natriumsulfid und Schwefel im Reaktionsgemisch so wie oben erreicht wird.

In einer weiteren Ausführungsform betrifft die vorliegende Erfindung auch ein Kit, das die erfindungsgemäße Lösung enthält. Die Lösung kann in einem oder mehr Behältern abgepackt sein. Die Bestandteile der erfindungsgemäßen Lösung, insbesondere Natriumdisulfid, vorzugsweise als Trihydrat, Schwefel und Methanol oder eine methanolische Lösung, können getrennt oder zusammen in einem Behälter des Kits abgepackt sein. Das Kit kann für die Durchführung des erfindungsgemäßen Verfahrens eingesetzt werden und kann Anweisungen für die Durchführung enthalten.

Verschiedene Dokumente sind in dem vorliegenden Text dieser Beschreibung zitiert. Jedes der Dokumente (einschließlich von Anleitungen und Beschreibungen von Herstellern) gilt hiermit durch die Referenz als von der Beschreibung mit umfasst. Dies heißt jedoch nicht, dass jedes der genannten Dokumente tatsächlich Stand der Technik für die vorliegende Erfindung ist.

Die vorliegende Erfindung wird durch die folgenden Beispiele verdeutlicht, ohne dass diese in irgendeiner Weise als einschränkend gelten.

### Beispiele

### Beispiel 1: Synthese des 6,8-Bis-methansulfonyloxyoctanoat

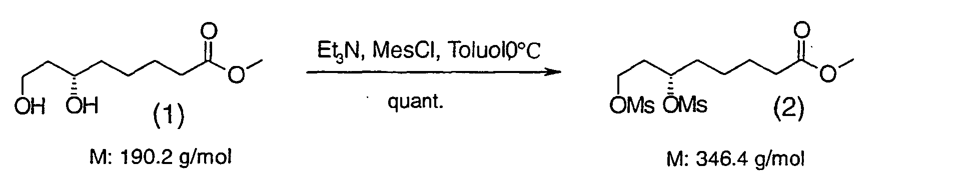

In einem 2-L HWS Gefäß wurden 98 g (0.50 mol) 6,8 Dihydroxioctansäuremethylester bei RT in 1500 ml Toluol vorgelegt. Der Ansatz wurde auf 0°C gekühlt und mit 173 ml (1.25mol) Triethylamin versetzt. Innerhalb von 2 Stunden wurden bei 0-5°C Innentemperatur 143,2 g (1,25 mol) Methansulfonsäurechlorid zugetropft. Der Ansatz wurde dann auf 25°C erwärmt und zwei Stunden nachgerührt.

Zur Abtrennung des Triethylaminhydrochlorids gab man 300 g Eiswasser zur Reaktionsmischung und rührte 5 Minuten intensiv nach. Die wässrige Phase wurde abgetrennt und einmal mit Toluol extrahiert.

Die vereinigten Toluolphasen wurden einmal mit VE-Wasser gewaschen und eingedampft (Druck: 60 ä 30 mbar, Manteltemperatur: 50°C, Innentemperatur.: < 45°C). Die Rohlösung wird direkt in die nächste Stufe eingesetzt.
- Ausbeute:: 268,3 g Rohlösung (Umsatz: quantitativ)

### Beispiel 2: Schwefeleinführung

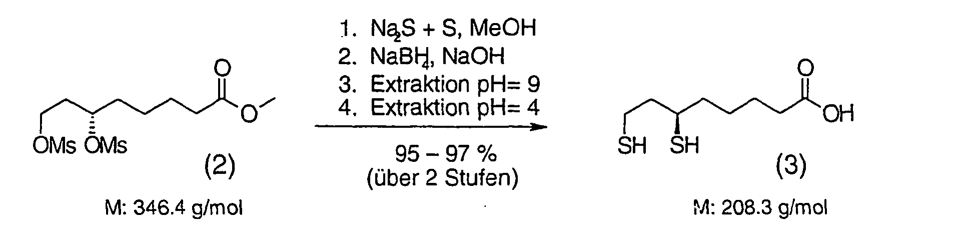

0,5 mol der Bismesylatlösung wurden mit Methanol verdünnt und auf 40°C erwärmt. Innerhalb von 4 Std. tropfte man unter N₂-Atmosphäre eine Lösung aus 250 ml Methanol, 81,8 g (0,65 mol) Na₂S • 3 H₂O und 24,0 g (0,75 mol) Schwefel bei 40°C zu und ließ weitere drei Stunden rühren.

Man gibt 1000 ml VE-Wasser und anschließend 0,65 mol Borollösung zu, destilliert ab und rührt bei dieser Temperatur noch zwei Stunden nach.

Der Ansatz wird mit 100 ml Toluol versetzt und mit H₂SO₄ auf pH 9 gestellt. Die Toluolphase wird abgetrennt und verworfen.

Die wässrige Phase wird mit Toluol versetzt und mit H₂SO₄ auf pH 4,5 gestellt. Nach Phasentrennung wird die wässrige Phase einmal mit Toluol nachextrahiert. Die vereingten toluolischen Phasen werden mit VE-Wasser gewaschen und anschließend im Vakuum eingedampft.
- Ausbeute:: 125,7 g (96,6 % bez. Diol 1)
- Gehalt (GC i. St.):: 78 % R-Dihydroliponsäure, 1,8 % R-Liponsäure

### Beispiel 3: Oxidation zur Liponsäure

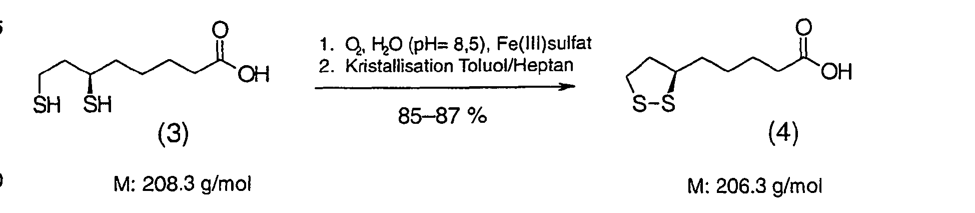

In einen 10 L Rundkolben werden 125,5 g der Dihydroliponsäure-Lösung in 5 L VE-Wasser aufgerührt, die Lösung mit verdünnter Natronlauge auf pH 8,5 gestellt und mit katalytischen Mengen Fe(III)sulfat-Lösung versetzt. Bis zur Farbaufhellung wird mit Luft begast. Nach Zugabe von 600 ml Toluol wird mit H₂SO₄ auf pH 2 gestellt. Man trennt die Phasen und extrahiert die Wasserphase einmal mit Toluol. Die vereinigten Toluolphasen werden auf ca. 25 % des Volumens eingeengt. Der Rückstand wurde mit 600 ml Heptan versetzt, unter Stickstoff gerührt und anschließend über ein mit Kieselgel beladenes Filter gedrückt. Das Filter wird mit Toluol/Heptan-Gemisch nachgewaschen.

Die vereinigten Filtrate werden im 2 L HWS-Gefäß bei Raumtemperatur angeimpft. Der Ansatz wird abgekühlt und anschließend bei -10°C nachgerührt. Die gelben Kristalle werden abfiltriert, mit Heptan gewaschen und bis zur Gewichtskonstanz getrocknet.
- Ausbeute:: 85,6 % bez. auf Diol 1
- Gehalt (GC i. St.):: 99,95 %
- ee-Wert:: > 99 %, das S-Enantiomer konnte nicht detektiert werden

In der Mutterlauge konnten zusätzlich 8,2 % R-Liponsäure nachgewiesen werden.

### Beispiel 4

(a) (1 → 2): 170 ml (1,25 mol) Triethylamin und eine Lösung von 98 g (0,5 mol) (6S)-6,8-Dihydroxyoctansäuremethylester 1 werden in 1 Liter Toluol vorgelegt. Man kühlt ab und gibt 143 g (1,25 mol) Mesylchlorid zu. Nach Abtrennung des Triethylammonium-hydrochlorids engt man die Lösung ein. Der Umsatz ist quantitativ.

(b) (2 → 3): 151 g (0,63 mol) Natriumsulfid und 24 g Schwefelpulver werden in Methanol aufgekocht. Die Reaktionsmischung wird mit 0,5 mol des Mesylats versetzt. Man verdünnt mit voll-entsalzten Wasser (VE-Wasser). Nach Reaktion mit 174 g (0,55 mol) 12 % NaBH₄-Lösung in 14 M Natronlauge (Borol-Lösung) destilliert man das Lösungsmittel ab. Der Ansatz wird auf pH 1 gestellt und mit Toluol extrahiert. Ausbeute: 105,1 g (90 %, 91 % bzgl. Diol 1)

(c) (3 → 4): In einen 10-Liter-Rundkolben werden 105,1 g Dihydroliponsäure in 5 Liter VE-Wasser aufgerührt, die Lösung auf pH 8,5 gestellt und mit katalytischen Mengen Fe(III)chlorid versetzt. Bis zum vollständigen Umsatz wird mit Luft begast. Die Lösung wird auf pH 2 gestellt und mit Toluol extrahiert. Die Phasen werden getrennt und die organische Phase eingeengt. Der Rückstand wird mit technischem Heptan versetzt und über ein mit 5 g Kieselgel beladenes Filter gedrückt.

Unter Kühlung kristallisiert R-Liponsäure aus, die im Stickstoffstrom getrocknet wird.
Die Ausbeute beträgt 65,9 g (64 % d. Th. bzgl. Diol 1).
GC-Gehalt: > 99,9 %
ee-Gehalt: > 99 %

### Beispiel 5: Einführung der Destillation

(a) (1 → 2): 170 ml (1,25 mol) Triethylamin und eine Lösung von 98 g (0,5 mol) (6S)-6,8-Dihydroxyoctansäuremethylester 1 werden in 1 Liter Toluol vorgelegt. Man kühlt ab und gibt 143 g (1,25 mol) Mesylchlorid zu. Nach Abtrennung des Triethylammonium-hydrochlorids engt man die Lösung ein. Der Umsatz ist quantitativ.
(b) (2 → 3): 151 g (0,63 mol) Natriumsulfid und 24 g Schwefelpulver werden in Methanol aufgekocht. Die Reaktionsmischung wird mit 0,5 mol des Mesylats versetzt. Man verdünnt mit VE-Wasser, gibt 174 g (0,55 mol) Borol-Lösung zu und destilliert das Lösungsmittel ab. Der Ansatz wird auf pH 1 gestellt und mit Toluol extrahiert. Die organische Phase wird vom Lösungsmittel befreit. Das zurückbleibende Öl wird im Fallfilmverdampfer destilliert (1 bis 3 mbar, 200°C). Ausbeute: 95,3 g (96 %ig, 88 % bzgl. Diol 1).
(c) (3 → 4): In einen 10-Liter-Rundkolben werden 95,3 g destillierte Dihydroliponsäure in 5 Liter VE-Wasser aufgerührt, die Lösung auf pH 8,5 gestellt und mit katalytischen Mengen Fe(III)chlorid versetzt. Bis zum vollständigen Umsatz wird mit Luft begast. Die Lösung wird auf pH 2 gestellt und mit Toluol extrahiert. Die Phasen werden getrennt und die organische Phase eingeengt. Der Rückstand wird mit technischem Heptan versetzt und über ein mit 5 g Kieselgel beladenes Filter gedrückt.
   Unter Kühlung kristallisiert R-Liponsäure aus, die im
   Stickstoffstrom getrocknet wird.
   Die Ausbeute beträgt 74,2 g (72 % d. Th. bzgl. Diol 1).
   GC-Gehalt: > 99,9 %
   ee-Gehalt: > 99 %

### Beispiel 6: Extraktion bei pH 9 und Destillation

(a) (1 → 2): 170 ml (1,25 mol) Triethylamin und eine Lösung von 98 g (0,5 mol) (6S)-6,8-Dihydroxyoctansäuremethylester 1 werden in 1 Liter Toluol vorgelegt. Man kühlt ab und gibt 143 g (1,25 mol) Mesylchlorid zu. Nach Abtrennung des Triethylammonium-hydrochlorids engt man die Lösung ein. Der Umsatz ist quantitativ.
(b) (2 → 3): 151 g (0,63 mol) Natriumsulfid und 24 g Schwefelpulver werden in Methanol aufgekocht. Die Reaktionsmischung wird mit 0,5 mol des Mesylats versetzt. Man verdünnt mit VE-Wasser und gibt 174 g (0,55 mol) Borol-Lösung zu. Der Ansatz wird mit Schwefelsäure auf pH 9 gestellt und mit Toluol extrahiert. Die Toluolphase wird verworfen. Der Ansatz wird anschließend auf pH 1 gestellt und mit Toluol extrahiert. Die organische Phase wird vom Lösungsmittel befreit. Das zurückbleibende Öl wird im Fallfilmverdampfer destilliert (1 bis 3 mbar, 200°C).
   Ausbeute: 91,1 g (95 %, 85 % bzgl. Diol 1).
(c) (3 → 4): In einen 10-Liter-Rundkolben werden 91,1 g destillierte Dihydroliponsäure in 5 Liter VE-Wasser aufgerührt, die Lösung auf pH 8,5 gestellt und mit katalytischen Mengen Fe(III)chlorid versetzt. Bis zum vollständigen Umsatz wird mit Luft begast. Die Lösung wird auf pH 2 gestellt und mit Toluol extrahiert. Die Phasen werden getrennt und die organische Phase eingeengt. Der Rückstand wird mit technischem Heptan versetzt und über ein mit 5 g Kieselgel beladenes Filter gedrückt.
   Unter Kühlung kristallisiert R-Liponsäure aus, die im
   Stickstoffstrom getrocknet wird.
   Die Ausbeute beträgt 76,2 g (74 % d. Th. bzgl. Diol 1)
   GC-Gehalt: > 99,9 %
   ee-Gehalt: > 99 %

### Beispiel 7: Extraktionen bei pH 9, pH 4 und Destillation

(a) (1 → 2): 170 ml (1,25 mol) Triethylamin und eine Lösung von 98 g (0,5 mol) (6S)-6,8-Dihydroxyoctansäuremethylester 1 werden in 1 Liter Toluol vorgelegt. Man kühlt ab und gibt 143 g (1,25 mol) Mesylchlorid zu. Nach Abtrennung des Triethylammonium-hydrochlorids engt man die Lösung ein. Der Umsatz ist quantitativ.
(b) (2 → 3): 151 g (0,63 mol) Natriumsulfid und 24 g Schwefelpulver werden in Methanol aufgekocht. Die Reaktionsmischung wird mit 0,5 mol des Mesylats versetzt. Man verdünnt mit VE-Wasser und gibt 174 g (0,55 mol) Borol-Lösung zu. Der Ansatz wird mit Schwefelsäure auf pH 9 gestellt und mit Toluol extrahiert. Die Toluolphase wird verworfen. Der Ansatz wird anschließend auf pH 4 gestellt und mit Toluol extrahiert. Die organische Phase wird vom Lösungsmittel befreit. Das zurückbleibende Ö1 wird im Fallfilmverdampfer destilliert (1 bis 3 mbar, 200°C).
   Ausbeute: 95,2 g (97 %, 88 % bzgl. Diol 1).
(c) (3 → 4): In einen 10-Liter-Rundkolben werden 95,2 g destillierte Dihydroliponsäure in 5 Liter VE-Wasser aufgerührt, die Lösung auf pH 8,5 gestellt und mit katalytischen Mengen Fe(III)chlorid versetzt. Bis zum vollständigen Umsatz wird mit Luft begast. Die Lösung wird auf pH 2 gestellt und mit Toluol extrahiert. Die Phasen werden getrennt und die organische Phase eingeengt. Der Rückstand wird mit technischem Heptan versetzt und über ein mit 5 g Kieselgel beladenes Filter gedrückt.
   Unter Kühlung kristallisiert R-Liponsäure aus, die im
   Stickstoffstrom getrocknet wird.
   Die Ausbeute beträgt 77,2 g (75 % d. Th. bzgl. Diol 1)
   GC-Gehalt: > 99,9 %
   ee-Gehalt: > 99 %

### Beispiel 8: Extraktionen bei pH 4, pH 9

(a) (1 → 2): 170 ml (1,25 mol) Triethylamin und eine Lösung von 98 g (97 %, 0,5 mol) (6S)-6,8-Dihydroxyoctansäuremethylester 1 werden in 1 Liter Toluol vorgelegt. Man kühlt ab und gibt 143 g (1,25 mol) Mesylchlorid zu. Nach Abtrennung des Triethylammonium-hydrochlorids engt man die Lösung ein. Der Umsatz ist quantitativ.
(b) (2 → 3): 151 g (0,63 mol) Natriumsulfid und 24 g Schwefelpulver werden in Methanol aufgekocht. Die Reaktionsmischung wird mit 0,5 mol des Mesylats versetzt. Man verdünnt mit VE-Wasser und gibt 174 g (0,55 mol) Borol-Lösung zu. Der Ansatz wird mit Schwefelsäure auf pH 4 gestellt und mit Toluol extrahiert. Die wäßrige Phase wird verworfen. Der Ansatz wird anschließend auf pH 9 gestellt und mit Toluol extrahiert. Die organische Phase wird verworfen.
(c) (3 → 4): Die erhaltene wässrige Lösung wird mit VE-Wasser auf 5 Liter aufgerührt und mit katalytischen Mengen Fe(III)chlorid versetzt. Bis zum vollständigen Umsatz wird mit Luft begast. Die Lösung wird auf pH 2 gestellt und mit Toluol extrahiert. Die Phasen werden getrennt und die organische Phase eingeengt. Der Rückstand wird mit technischem Heptan versetzt und über ein mit 5 g Kieselgel beladenes Filter gedrückt.
   Unter Kühlung kristallisiert R-Liponsäure aus, die im
   Stickstoffstrom getrocknet wird.
   Die Ausbeute beträgt 73 % d. Th. bzgl. Diol 1
   GC-Gehalt: > 99,9 %
   ee-Gehalt: > 99 %

### Beispiel 9: Schwefeleinführung

Gemäß den Verfahren, wie sie in den Beispielen 1 bis 8 beschrieben wurden, kann Schwefel auch in Mesylate von Verbindungen, die das Strukturelement (3) enthalten, eingeführt werden.

Folgende Tabelle zeigt, dass nach dem erfindungsgemäßen Verfahren Disulfide in verschiedene Diole, zu Synthese von 4er, 5er oder 6er-Ringen mit guten Ausbeuten eingeführt werden kann. Ebenso wurde mit 2,4-Pentandiol ein guter Umsatz bei der Mesylierung und bei der Schwefeleinführung erreicht.

**Tabelle**

| Diol | Mesylat Ausbeute | Disulfid Ausbeute |
|---|---|---|
| 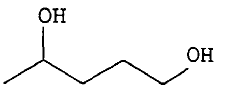 | 98 % | 58 % |
| 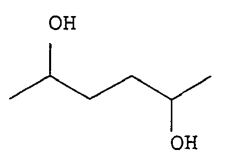 | 76 % | 40 % |
| 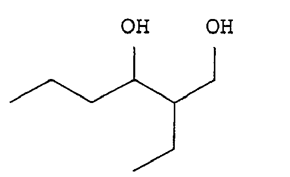 | 100 % | 85 % |
| 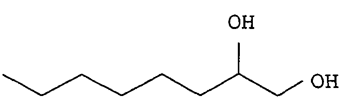 | 100 % | 56 % |

## Patentansprüche

1. Verfahren zur Herstellung von R- oder S-Dihydroliponsäure umfassend
(a) Umsetzung von 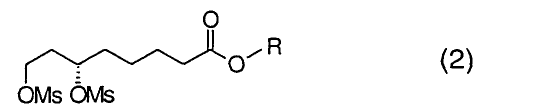
oder dessen Stereoisomer,
wobei
MS für SO₂-R' steht und R und R' unabhängig voneinander C₁-C₆-Alkyl, C₃-C₈-Cycloalkyl, C₃-C₈-Cycloalkylalkyl, Aryl oder Aralkyl bedeutet,
mit Natriumsulfid und Schwefel in Methanol.

2. Verfahren zur Herstellung von Verbindungen das Strukturelement (3) 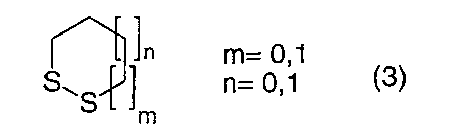 enthaltend, umfassend die
(a) Umsetzung von (4) 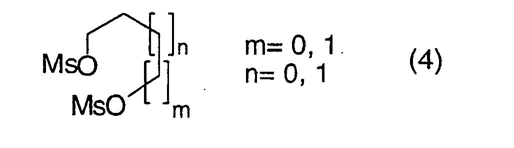
mit Natriumsulfid-Trihydrat und Schwefel in Methanol.

3. Verfahren nach Anspruch 1, wobei die Umsetzung mit Natriumsulfid-Trihydrat durchgeführt wird.

4. Verfahren nach einem der Ansprüche 1 bis 3, wobei Schwefel in einem molaren Überschuss über dem Natriumsulfid vorliegt.

5. Verfahren nach einem der Ansprüche 1 bis 4, umfassend mindestens einen weiteren anschließenden Verfahrensschritt, ausgewählt aus der Gruppe bestehend aus:
(b) Umsetzung mit einem Komplexhydrid;
(c) Extraktion einer protischen Lösung R-Dihydroliponsäure oder S-Dihydroliponsäure mit organischen Lösungsmitteln bei einem pH-Wert von 9 bis 10;
(d) Extraktion von R-Dihydroliponsäure oder S-Dihydroliponsäure mit organischen Lösungsmitteln aus einer protischen Lösung bei einem pH-Wert von 4 bis 5;
und
(e) Destillation der Dihydroliponsäure.

6. Verfahren nach einem der Ansprüche 1 bis 5, wobei Natriumsulfid-Trihydrat und Schwefeln in Methanol zu (2) oder (4) zugegeben wird.

7. Verfahren nach einem der Ansprüche 1 bis 6, wobei die Reaktionstemperatur zwischen Raumtemperatur und 70°C liegt.

8. Verfahren nach Anspruch 7, wobei die Reaktionstemperatur zwischen 35 und 45°C liegt.

9. Verfahren zur Herstellung von R-Liponsäure oder S-Liponsäure umfassend die Verfahrensschritte nach einem der Schritte 1 bis 8 und
(f) anschließende Oxidation der R oder S-Dihydroliponsäure.

10. Verfahren zur Herstellung R-Dihydroliponsäure oder S-Dihydroliponsäure umfassend das Verfahren nach einem der Ansprüche 1 bis 9, wobei die R-Dihydroliponsäure oder S-Dihydroliponsäure chemisch rein ist.

11. Verfahren nach einem der Ansprüche 1 bis 10, umfassend die Weiterverarbeitung von R-Liponsäure oder S-Liponsäure in pharmakologisch verträgliche Salze oder Derivate.

12. Verfahren zur Herstellung Kosmetika, eines Arzneimittels, oder Pharmakons, umfassend einen der Schritte von Anspruch 1 bis 11 und,
(g) Formulierung der R-Dihydroliponsäure, S-Dihydroliponsäure, R-Liponsäure oder S-Liponsäure in einer dermatologisch oder pharmakologisch verträglichen Form.

13. Lösung, enthaltend Natriumsulfid-Trihydrat und Schwefel in Methanol, wobei der Schwefel in einem molaren Überschuss vorliegt.

14. Lösung nach Anspruch 12, wobei der molare Schwefelüberschuss über dem Natriumsulfid-Trihydrat zwischen 5 und 30 % liegt.

15. Kit, die Lösung nach Anspruch 13 oder 14 umfassend.

## Claims

1. A process for the preparation of R- or S-dihydrolipoic acid comprising
(a) reaction of 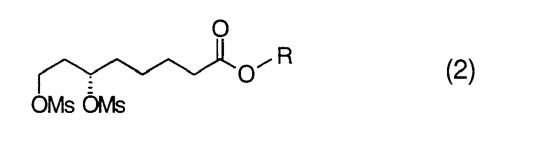
or its stereoisomer,
where
MS is SO₂-R' and R and R' independently of one another are C₁-C₆-alkyl, C₃-C₈-cycloalkyl, C₃-C₈-cycloalkylalkyl, aryl or aralkyl,
with sodium sulfide and sulfur in methanol.

2. A process for the preparation of compounds comprising the structural element (3) 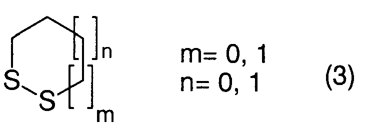 comprising the
(a) reaction of (4) 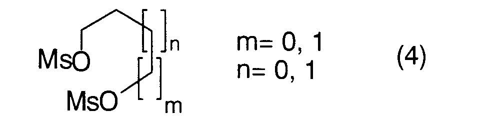
with sodium sulfide trihydrate and sulfur in methanol.

3. The process according to claim 1, the reaction being carried out with sodium sulfide trihydrate.

4. The process according to one of claims 1 to 3, sulfur being present in a molar excess over the sodium sulfide.

5. The process according to one of claims 1 to 4, comprising at least one further subsequent process step selected from the group consisting of:
(b) reaction with a complex hydride;
(c) extraction of a protic solution of R-dihydrolipoic acid or S-dihydrolipoic acid with organic solvents at a pH of 9 to 10;
(d) extraction of R-dihydrolipoic acid or S-dihydrolipoic acid with organic solvents from a protic solution at a pH of 4 to 5;
and
(e) distillation of the dihydrolipoic acid.

6. The process according to one of claims 1 to 5, sodium sulfide trihydrate and sulfur in methanol being added to (2) or (4).

7. The process according to one of claims 1 to 6, the reaction temperature being between room temperature and 70°C.

8. The process according to claim 7, the reaction temperature being between 35 and 45°C.

9. A process for the preparation of R-lipoic acid or S-lipoic acid comprising the process steps according to any one of claims 1 to 8 and
(f) subsequent oxidation of the R- or S-dihydrolipoic acid.

10. A process for the preparation of R-dihydrolipoic acid or S-dihydrolipoic acid comprising the process according to one of claims 1 to 9, the R-dihydrolipoic acid or S-dihydrolipoic acid being chemically pure.

11. The process according to one of claims 1 to 10, comprising the further processing of R-lipoic acid or S-lipoic acid into pharmacologically tolerable salts or derivatives.

12. The process for the production of cosmetics, of a pharmaceutical or pharmacon, comprising one of the steps of claims 1 to 11, and
(g) formulation of the R-dihydrolipoic acid, S-dihydrolipoic acid, R-lipoic acid or S-lipoic acid in a dermatologically or pharmacologically tolerable form.

13. A solution comprising sodium sulfide trihydrate and sulfur in methanol, the sulfur being present in a molar excess.

14. The solution according to claim 13, the molar excess of sulfur over the sodium sulfide trihydrate being between 5 and 30%.

15. A kit comprising the solution according to claim 13 or 14.

## Revendications

1. Procédé de préparation d'acide R-dihydrolipoïque ou S-dihydrolipoïque comprenant
(a) une réaction de 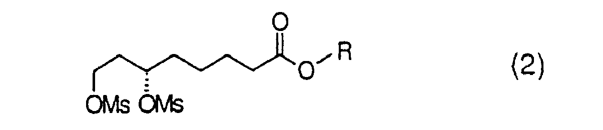
ou de son stéréoisomère,
où
Ms représente SO₂-R' et R et R' représentent, indépendamment l'un de l'autre, un groupe alkyle en C₁-C₆, cycloalkyle en C₃-C₈, cycloalkylalkyle en C₃-C₈, aryle ou aralkyle,
avec du sulfure de sodium et du soufre dans du méthanol.

2. Procédé de préparation de composés contenant l'élément structurel (3) : 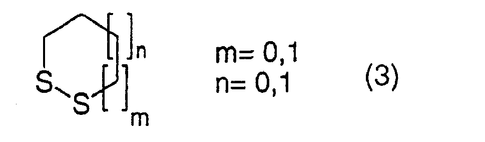 comprenant
(a) la réaction de (4) : 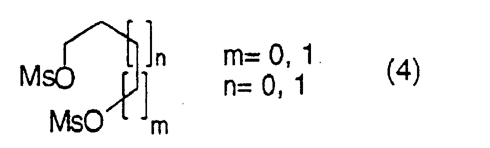
avec du trihydrate de sulfure de sodium et du soufre dans du méthanol.

3. Procédé suivant la revendication 1, dans lequel la réaction est effectuée avec du trihydrate de sulfure de sodium.

4. Procédé suivant l'une des revendications 1 à 3, dans lequel le soufre se présente en un excès molaire par rapport au sulfure de sodium.

5. Procédé suivant l'une des revendications 1 à 4, comprenant au moins une autre étape ultérieure, choisie parmi le groupe constitué
(b) d'une réaction avec un hydrure complexe,
(c) d'une extraction d'une solution protique d'acide R-dihydrolipoïque ou d'acide S-dihydrolipoïque avec des solvants organiques, à une valeur de pH de 9 à 10,
(d) d'une extraction d'acide R-dihydrolipoïque ou d'acide S-dihydrolipoïque avec des solvants organiques à partir d'une solution protique, à une valeur de pH de 4 à 5,
et
(e) d'une distillation de l'acide dihydrolipoïque.

6. Procédé suivant l'une des revendications 1 à 5, dans lequel du trihydrate de sulfure de sodium et du soufre dans du méthanol sont ajoutés à (2) ou (4) .

7. Procédé suivant l'une des revendications 1 à 6, dans lequel la température réactionnelle est située entre la température ambiante et 70°C.

8. Procédé suivant la revendication 7, dans lequel la température réactionnelle est située entre 35 et 45°C.

9. Procédé de préparation d'acide R-lipoïque ou d'acide S-lipoïque comprenant les étapes suivant une des revendications 1 à 8, et
(f) une oxydation ultérieure de l'acide R-dihydrolipoïque ou S-dihydrolipoïque.

10. Procédé de préparation d'acide R-dihydrolipoïque ou d'acide S-dihydrolipoïque comprenant le procédé suivant l'une des revendications 1 à 9, dans lequel l'acide R-dihydrolipoïque ou l'acide S-dihydrolipoïque est chimiquement pur.

11. Procédé suivant l'une des revendications 1 à 10, comprenant la poursuite du traitement d'acide R-lipoïque ou d'acide S-lipoïque en sels ou dérivés pharmacologiquement compatibles.

12. Procédé de préparation d'un produit cosmétique, d'un médicament ou d'un produit pharmaceutique, comprenant une des étapes des revendications 1 à 11, et
(g) une formulation de l'acide R-dihydrolipoïque, de l'acide S-dihydrolipoïque, de l'acide R-lipoïque ou de l'acide S-lipoïque sous une forme dermatologiquement ou pharmacologiquement compatible.

13. Solution contenant du trihydrate de sulfure de sodium et du soufre dans du méthanol, dans laquelle le soufre se présente en un excès molaire.

14. Solution suivant la revendication 13, dans laquelle l'excès molaire en soufre par rapport au trihydrate de sulfure de sodium est compris entre 5 et 30 %.

15. Trousse qui comporte une solution suivant la revendication 13 ou 14.
